# EUROPÄISCHE PATENTSCHRIFT

(11) **EP 0 947 206 B1**
(45) Veröffentlichungstag und Bekanntmachung des Hinweises auf die Patenterteilung: **04.04.2007**
(21) Anmeldenummer: 99103679.9
(22) Anmeldetag: 25.02.1999
(51) Int. Cl.: A61M 1/16

(54) **Dialysegerät mit Vorrichtung zur Herstellung von Dialyselösungen**
Dialysis system with apparatus for preparing dialysates
Système de dialyse avec appareil d'élaboration de dialysates

(30) Priorität: 01.04.1998 DE 19814687
(43) Veröffentlichungstag der Anmeldung: 06.10.1999
(60) Teilanmeldung: 04012755.7
(73) Patentinhaber: Fresenius Medical Care Deutschland GmbH, 61352 Bad Homburg (DE)
(72) Erfinder: Dönig, Rainer, 60489 Frankfurt/M. (DE); Döpper, Joachim, 64521 Gross-Gerau (DE); Schulz, Wolfgang, 66606 St. Wendel (DE)
(74) Vertreter: Laufhütte, Dieter

(56) Entgegenhaltungen:
- EP-A- 0 311 848
- EP-A- 0 575 970
- WO-A-96/25214

## Beschreibung

Die vorliegende Erfindung betrifft ein Dialysegerät mit einer Vorrichtung zur Herstellung von Dialyselösungen.

Ein gattungsgemäßes Dialysegerät ist beispielsweise aus der WO 92/11046 bekannt. Hier wird ein Dialysegerät mit einer Vorrichtung offenbart, mit der Dialyselösungen gewünschter Beschaffenheit hergestellt und in den Dialyseflüssigkeitskreislauf des Gerätes eingespeist werden. Zur Herstellung der benötigten Lösungen werden Tabletten verwendet, die die erforderlichen Bestandteile der Dialyselösungen enthalten und die bei Bedarf in eine wasserenthaltende Mischkammer eingegeben und darin in Wasser gelöst werden. Die Tabletten können beispielsweise in einem über der Mischvorrichtung angeordneten Magazin vorgesehen sein. Ein den Bedürfnissen der Patienten angepaßter Konzentrationsverlauf der chemischen Bestandteile der Dialyselösungen wird dadurch erreicht, daß zu vorgebbaren Zeitpunkten unterschiedliche Wirkstoffmengen oder -arten enthaltende Tabletten dem Lösevorgang und anschließend dem Dialyseflüssigkeitskreislauf zugeführt werden. Um Fehler bei der Art und Konzentration der verabreichten Dialyselösungen zu vermeiden und um dem Prozeß überwachen zu können, weisen die Tabletten einen Strichcode auf, der mittels einer Lesevorrichtung des Dialysegerätes erfaßt werden kann. Ein Nachteil dieser Vorrichtung besteht darin, daß entsprechend der Breite des anzuwendenden Konzentrationsbereichs eine Vielzahl unterschiedliche Wirkstoffkonzentrationen enthaltende Tabletten hergestellt und mit einem Strichcode versehen werden müssen, was deren Herstellung entsprechend aufwendig und teuer gestaltet. Zudem sind aufgrund der festen Beschaffenheit der Tabletten Vorrichtungen zur Zugabe der Tabletten in ein geeignetes Lösemittel sowie zum Lösen der Tabletten notwendig, wodurch die Dialysegeräte einen verhältnismäßig komplexen Aufbau annehmen.

WO-A-96 25214 offenbart eine Vorrichtung zur Herstellung von Dialyselösungen, bei der die Vorratsflaschen mit Kennzeichnungsmitteln versehen sind, welche durch eine Erfassungsvorrichtung detektierbar sind.

Es ist die Aufgabe der vorliegenden Erfindung ein Dialysegerät zur Verfügung zu stellen, mit dem auf einfache Weise unterschiedlich konzentrierte Dialyseflüssigkeiten zuverlässig herstellbar sind.

Diese Aufgabe wird durch ein Dialysegerät gemäß Anspruch 1 gelöst. Die Vorrichtung umfaßt eine Erfassungsvorrichtung, wenigstens zwei Anschlüsse sowie wenigstens zwei austauschbare Vorratsbehälter zur Aufnahme der zu dosierenden Lösungsbestandteile, die jeweils mit mindestens einem Konnektor verbunden sind, wobei die Konnektoren mit den Anschlüssen verbindbar sind, und wobei die Konnektoren oder den Konnektoren benachbarte Bereiche eines Verbindungsschlauches Kennzeichnungsmittel aufweisen, die mittels der Erfassungsvorrichtung detektierbar sind. Dadurch wird es möglich, einfach und zuverlässig unterschiedlich konzentrierte Dialyselösungen herzustellen, wobei als Ausgangslösungen übliche Standardlösungen eingesetzt werden können. Entsprechend entfällt die Notwendigkeit, zahlreiche unterschiedlich konzentrierte Standardlösungen bereitzustellen, um eine den Bedürfnissen des Patienten optimal angepaßte Dialyse durchführen zu können.

Erfindungsgemäß weisen die Konnektoren Kennzeichnungsmittel auf, die mittels der Erfassungsvorrichtung detektierbar sind. Dadurch werden Fehler bei der Zuordnung der Vorratsbehälter zu den Anschlüssen der Vorrichtung sicher vermieden. Aufgrund der die Vorratsbehälter identifizierenden Kennzeichnungsmittel erfaßt das Dialysegerät automatisch die Art und/oder Menge der enthaltenen Lösung, so daß unabhängig von der Wahl des Anschlusses stets eine eindeutige Identifizierung erfolgt.

Der Bediener des Dialysegerätes steckt in beliebiger Reihenfolge die Konnektoren der benötigten Vorratsbehälter auf die Anschlüsse der Vorrichtung und gibt über eine Steuereinrichtung die gewünschten Konzentrationen bzw. Konzentrationsgradienten der herzustellenden Dialyseflüssigkeit ein. Das erfindungsgemäße Dialysegerät erkennt anhand der Kennzeichnungsmittel selbständig welcher Anschluß mit welchen Lösungen beaufschlagt wird und führt entsprechend beispielsweise durch die Schaltung von Pumpen oder Ventilen den gewünschten Herstell- bzw. Mischvorgang durch. Eine möglicherweise zur Gefährdung des Patienten führende Verwechslung von Anschlüssen und somit die Herstellung von Dialyselösungen mit unerwünschten Wirkstoffen oder Wirkstoffmengen kann somit sicher vermieden werden. Ein weiterer Vorteil der erfindungsgemäßen Vorrichtung ergibt sich daraus, daß auch zur Herstellung zahlreicher unterschiedlich konzentrierter Dialyseflüssigkeiten stets nur eine geringe Anzahl von Standardlösungen notwendig ist, was produktionstechnisch und logistisch zu erheblichen Vereinfachungen führt.

Gemäß einer bevorzugten Ausgestaltung der vorliegenden Erfindung sind als Vorratsbehälter Lösungsbeutel vorgesehen, wobei die Lösungsbeutel einen Verbindungsschlauch umfassen, an dessen Ende ein Konnektor vorgesehen ist. Die Lösungsbeutel enthalten beispielsweise Standardlösungen, die nur in geringer Anzahl zur Verfügung gestellt werden müssen.

Das Kennzeichnungsmittel kann an einer vorgebbaren Position des Konnektors vorgesehen sein und die Erfassungsvorrichtung kann derart ausgeführt und/oder angeordnet sein, daß neben der Art auch die Position des Kennzeichnungsmittels erfaßbar ist. Die Schaffung des Kennzeichnungsmittels auf dem Konnektor hat den Vorteil, daß unabhängig von der Länge oder Anzahl der Verbindungsschläuche eine Verwechslung der Vorratsbehälter bzw. eine fehlerhafte Zuordnung zu den Anschlüssen ausgeschlossen ist. Vielmehr ist es unnötig, daß der Bediener darauf achtet, welcher Konnektor mit welchem Anschluß verbunden wird, da das erfindungsgemäße Dialysegerät eine automatische Erkennung des Konnektors und des damit in Verbindung stehenden Vorratsbehälters bzw. Lösungsbeutels durchführt. Es kann darüber hinaus vorgesehen sein, daß die Erfassungsvorrichtung die Position des Konnektors ermittelt, wodurch es dem Bediener möglich wird, zu erkennen, daß ein Konnektor nicht vollständig auf den Anschluß aufgesteckt ist. In diesem Fall wäre das Kennzeichnungsmittel gegenüber der vollständig aufgesteckten Position geringfügig verschoben, was durch die Erfassungsvorrichtung detektiert wird.

Besonders vorteilhaft ist es, wenn das Kennzeichnungsmittel einen Strichcode umfaßt. Derartige Codierungen sind in einer großen Vielzahl herstellbar und können problemlos auf die Konnektoren oder die Verbindungsschläuche aufgebracht werden. Darüber hinaus ermöglicht die Verwendung eines Strichcodes die Detektion nicht nur der Art der angeschlossenen Lösung, sondern auch die Erfassung einer Diskonnektion bzw. das Erkennen eines fehlerhaft aufgesteckten Konnektors.

Dabei kann vorgesehen sein, daß der Strichcode derart auf den Konnektoren angeordnet ist, daß die Streifen des Strichcodes in Umfangsrichtung des Konnektors verlaufen. Dadurch wird sichergestellt, daß ein Verdrehen des Konnektors nicht zu einer fehlenden oder mangelhaften Erfassung durch die Erfassungsvorrichtung führen kann, da sich der Strichcode über den gesamten Umfang erstreckt. Darüber hinaus ergibt sich der Vorteil, daß sich der Strichcode im Gegensatz zu in Längsrichtung des Konnektors verlaufenden Markierungen von der Erfassungsvorrichtung vollständig erfassen läßt.

Gemäß der vorliegenden Erfindung umfaßt das Kennzeichnungsmittel Informationen über Art und Volumen der zu dosierenden Lösungsbestandteile des Vorratsbehälters. Dadurch wird sichergestellt, daß nicht nur die Wirkstoffbeschaffenheit kontrollierbar ist, sondern auch der Zeitpunkt bestimmbar, ab dem möglicherweise ein Wechsel der Vorratsbehälter durchzuführen ist. Insbesondere ist es möglich, daß in diesem Fall die Erfassungsvorrichtung bereits zu Beginn der Behandlung ein für die anstehende Dialyse zu geringes Volumen der zu verabreichenden Lösung der Vorratsbehälter feststellt, was entsprechend dem Bediener angezeigt werden kann. Damit kann vermieden werden, daß eine Unterbrechung des Dialysevorganges dadurch notwendig wird, daß die Vorratsbehälter gewechselt werden müssen.

Gemäß einer bevorzugten Ausgestaltung der vorliegenden Erfindung ist eine Auswerteeinheit vorgesehen, die mit der Erfassungsvorrichtung verbindbar ist, wobei die Auswerteeinheit derart ausgeführt ist, daß Sollwerte für die Menge und/oder Beschaffenheit der in den Vorratsbehältern aufnehmbaren Lösungsbestandteilen der Dialyselösung speicherbar und mit den von der Erfassungsvorrichtung ermittelten Istwerten vergleichbar sind. Der Bediener des Dialysegerätes gibt vor Beginn der Behandlung die erforderlichen Daten beispielsweise für die gewünschten Wirkstoffkonzentrationen, -mengen und -gradienten ein, wonach diese Werte in der Auswerteeinheit als Sollwerte abgelegt werden. Anschließend werden die Vorratsbehälter mit den Anschlüssen der Vorrichtung mittels der Konnektoren verbunden und die Erfassungsvorrichtung ermittelt mit Hilfe der Kennzeichnungsmittel die Art und beispielsweise das Volumen der angeschlossenen Lösungen. Im Anschluß daran kann entsprechend der Vorgaben des Bedieners eine gewünschte Mischung bzw. ein zeitliches Profil von Wirkstoffkonzentrationen hergestellt werden, wenn die Auswerteeinheit keine Abweichungen zwischen den Vorgaben des Bedieners und den tatsächlich verwendeten Lösungen erkennt.

Besonders vorteilhaft ist es, wenn Signal- oder Absperrmittel vorgesehen sind, die mit der Auswerteeinheit verbindbar sind. Beispielsweise ist es möglich, daß beim unvollständigen Aufstecken eines Konnektors oder bei der Verwendung falscher Lösungsbestandteile ein optisches oder akustisches Signal ausgegeben wird oder daß Absperrmittel, wie z.B. Ventile, geschaltet werden, wodurch die Verabreichung der fehlerhaft angeschlossenen oder falschen Lösungen verhindert wird.

Die Absperrmittel können Mittel zur mechanischen und/oder elektrischen Absperrung von Leitungen umfassen. Erkennt die Auswerteeinheit eine Abweichung zwischen Soll- und Istwerten hinsichtlich der Art oder Menge der Wirkstoffe, werden die Absperrmittel derart betätigt, daß beispielsweise entweder eine Zufuhrleitung zum Dialysegerät abgesperrt wird, wodurch eine Abgabe von Dialyselösung in den Dialysator verhindert wird. Ebenso ist es möglich, daß die elektrische Versorgung beispielsweise der Pumpe des Dialysekreislaufes nicht aktivierbar ist, solange die Auswerteeinheit eine fehlende Übereinstimmung von Soll- und Istwerten feststellt.

Weitere Vorteile und Einzelheiten der vorliegenden Erfindung werden anhand eines in der Zeichnung dargestellten Ausführungsbeispiels näher erläutert. Es zeigt:

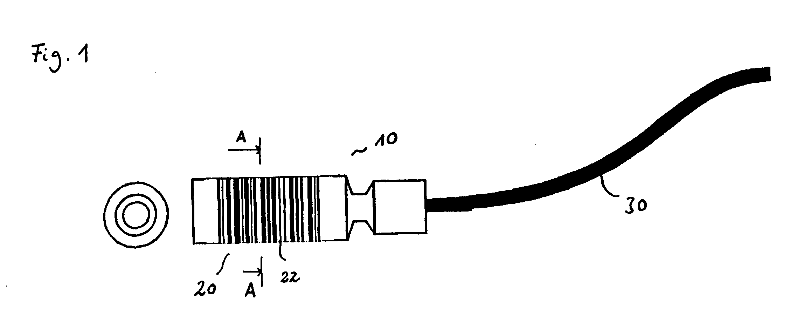

- Fig. 1:: Ein an einem Verbindungsschlauch zum Lösungsbeutel angeordneten Konnektor mit Strichcode.

Fig. 1 zeigt den Konnektor 10 in einer Schnittdarstellung (links) gemäß der Linie A-A der in Fig. 1 rechts dargestellten Seitenansicht. Der Konnektor 10 ist am Ende des Verbindungsschlauches 30 angeordnet, wobei der Verbindungsschlauch 30 an seinem anderen (nicht dargestellten) Ende mit einem die erforderlichen Lösungsbestandteile enthaltenden Lösungsbeutel verbunden ist.

Der Konnektor 10 weist als Kennzeichnungsmittel 20 den Strichcode 22 auf, wobei die Streifen des Strichcodes 22 in Umfangsrichtung des Konnektors 10 verlaufen. Dadurch wird sichergestellt, daß unabhängig davon, ob der Konnektor 10 beim Aufstecken auf einen Anschluß verdreht ist, eine Detektion des Strichcodes 22 durch die Erfassungsvorrichtung möglich ist.

Der Strichcode 22 enthält beispielsweise Informationen über die Art und Menge der Lösung, die in dem damit verbundenen Lösungsbeutel aufgenommen ist. Der Bediener des Dialysegerätes kann nach der Auswahl der geeigneten Lösungen die Konnektoren auf beliebige Anschlüsse der Dialysevorrichtung aufstecken, da erfindungsgemäß eine Erfassung der Art des Kennzeichnungsmittels 20 bzw. ein Lesen des Strichcodes 22 erfolgt. Aufgrund dieser Identifikation der Lösungsbeutel ist eine Verwechslung von Lösungen, die zur Abgabe fehlerhaft hergestellter Dialyseflüssigkeiten führen könnte, ausgeschlossen. Die Anordnung des Strichcodes 22 auf dem Konnektor 10 gemäß Fig. 1 ermöglicht es ferner, zu erkennen, ob der Konnektor 10 ordnungsgemäß und vollständig auf dem Anschluß der Vorrichtung aufgesteckt ist. Ist dies nicht der Fall, ermittelt die Erfassungsvorrichtung ein Streifenmuster, das dem Streifenmuster des vollständig aufgesteckten Konnektors 10 aufgrund der Verschiebung nicht entspricht. Dadurch wird das fehlerhafte Aufstecken sowie eine während des Betriebes erfolgende Diskonnektion sicher und zuverlässig erkannt.

Das erfindungsgemäße Dialysegerät ermöglicht es, eine erhöhte Behandlungsqualität dadurch zu erreichen, daß die verabreichten Dialyselösungen, insbesondere Peritonealdialyselösungen, individuell herstellbar und entsprechend für den Patienten optimal abstimmbar sind. Insbesondere können Standardlösungen eingesetzt werden, die in nur verhältnismäßig geringer Anzahl zur Verfügung gestellt werden müssen. Ein erhöhter Bedienungskomfort und eine höhere Betriebssicherheit wird dadurch erreicht wird, daß die Lösungsbeutel bzw. Vorratsbehälter in beliebiger Reihenfolge auf die Anschlüsse aufgesteckt werden können, da die eindeutige Identifikation der Lösungsbeutel bzw. deren Inhalt durch das erfindungsgemäße Gerät selbständig vorgenommen wird.

## Patentansprüche

1. Dialysegerät mit einer Vorrichtung zur Herstellung von Dialyselösungen, wobei die Vorrichtung wenigstens zwei Anschlüsse sowie wenigstens zwei austauschbare Vorratsbehälter zur Aufnahme der zu dosierenden Lösungsbestandteile umfasst, wobei die Vorratsbehälter mit mindestens einem Konnektor (10) verbunden sind, wobei die Konnektoren (10) mit den Anschlüssen verbindbar sind und wobei die Konnektoren (10) Kennzeichnungsmittel (20) aufweisen,
wobei die Vorrichtung eine Erfassungsvorrichtung umfasst, mittels derer die Kennzeichnungsmittel (20) detektierbar sind und mittels derer anhand der Kennzeichnungsmittel (20) eine automatische Erkennung des Konnektors (10) und des damit in Verbindung stehenden Vorratsbehälters durchführbar ist,
wobei die Kennzeichnungsmittel (20) Informationen über die Art und Volumen der zu dosierenden Lösungsbestandteile des Vorratsbehälters umfassen,
wobei die Vorrichtung eine Steuereinrichtung umfasst, in die Konzentrationen oder Konzentrationsgradienten der herzustellenden Dialyselösung eingebbar sind,
**dadurch gekennzeichnet, dass** die Vorratsbehälter über einen Verbindungsschlauch (30) jeweils mit dem mindestens einen Konnektor verbunden sind, wobei die Konnektoren in beliebiger Reihenfolge mit den Anschlüssen verbindbar sind und wobei die Vorrichtung Mittel umfasst, mittels derer auf der Grundlage der mit der Erfassungsvorrichtung detektierten Kennzeichnungsmittel (20) der Herstellvorgang der Dialyselösung durchführbar ist.

2. Dialysegerät nach Anspruch 1, **dadurch gekennzeichnet, daß** als Vorratsbehälter Lösungsbeutel vorgesehen sind und daß die Lösungsbeutel den Verbindungsschlauch (30) umfassen, an dessen Ende ein Konnektor (10) vorgesehen ist.

3. Dialysegerät nach Anspruch 1 oder 2, **dadurch gekennzeichnet, daß** das Kennzeichnungsmittel (20) einen Strichcode (22) umfaßt.

4. Dialysegerät nach einem oder mehreren der Ansprüche 1 bis 3, **dadurch gekennzeichnet, daß** eine Auswerteeinheit vorgesehen ist, die mit der Erfassungsvorrichtung verbindbar ist, wobei die Auswerteeinheit derart ausgeführt ist, daß Sollwerte für die Menge und/oder Beschaffenheit der in den Vorratsbehältern aufnehmbaren Lösungsbestandteile der Dialyselösung speicherbar und mit den von der Erfassungsvorrichtung ermittelten Istwerten vergleichbar sind.

5. Dialysegerät nach Anspruch 4, **dadurch gekennzeichnet, daß** Signal- oder Absperrmittel vorgesehen sind, die mit der Auswerteeinheit verbindbar sind.

6. Dialysegerät nach Anspruch 5, **dadurch gekennzeichnet, daß** die Absperrmittel Mittel zu mechanischen und/oder elektrischen Absperrung von Leitungen umfassen.

## Claims

1. A dialysis unit comprising an apparatus for the production of dialysis solutions, wherein the apparatus includes at least two connections and at least two replaceable storage containers for receiving the solution constituents to be metered, wherein the storage containers are connected to at least one connector (10), wherein the connectors (10) can be connected to the connections and wherein the connectors (10) have identification means (20), wherein the apparatus includes a detector device by means of which the identification means (20) can be detected and by means of which automatic recognition of the connector (10) and the storage container connected thereto can be effected on the basis of the identification means (20), wherein the identification means (20) include items of information about the nature and the volume of the solution constituents to be metered in the storage container, wherein the apparatus includes a control device into which levels of concentration or concentration gradients of the dialysis solution to be produced can be inputted,
**characterised in that** the storage containers are connected to the at least one connector by way of a respective connecting tube (30), wherein the connectors can be connected to the connections in any sequence and wherein the apparatus includes means, by means of which the dialysis solution production operation can be carried out on the basis of the identification means (20) detected with the detection device.

2. A dialysis unit according to claim 1 **characterised in that** solution bags are provided as the storage containers and that the solution bags include the connecting tube (30), at the end of which a connector (10) is provided.

3. A dialysis unit according to claim 1 or claim 2 **characterised in that** the identification means (20) includes a bar code (22).

4. A dialysis unit according to one or more of claims 1 to 3 **characterised in that** there is provided an evaluation unit which can be connected to the detection device, wherein the evaluation unit is designed in such a way that reference values for the amount and/or nature of the dialysis solution constituents which can be received in the storage containers can be stored and compared to the actual values ascertained by the detection device.

5. A dialysis unit according to claim 4 **characterised in that** there are provided signalling or cut-off means which can be connected to the evaluation unit.

6. A dialysis unit according to claim 5 **characterised in that** the cut-off means include means for mechanically and/or electrically cutting off lines.

## Revendications

1. Appareil de dialyse avec un dispositif pour l'élaboration de solutions de dialyse, sachant que le dispositif comprend au moins deux raccords, ainsi qu'au moins deux récipients de réserve interchangeables pour la réception des composants de solution à doser, sachant que les récipients de réserve sont reliés à au moins un connecteur (10), sachant que les connecteurs (10) peuvent être reliés aux raccords et sachant que les connecteurs (10) comportent des moyens de marquage (20), sachant que le dispositif comprend un dispositif de saisie au moyen duquel les moyens de marquage (20) peuvent être détectés et au moyen duquel, à l'aide des moyens de marquage (20), une reconnaissance automatique du connecteur (10) et du récipient de réserve avec lequel il est en communication est réalisable, sachant que les moyens de marquage (20) comprennent des informations sur la nature et le volume des composants à doser de la solution du récipient de réserve, sachant que le dispositif comprend un dispositif de commande dans lequel on peut entrer les concentrations ou les gradients de concentration de la solution de dialyse à préparer,
**caractérisé en ce que**
les récipients de réserve sont reliés chacun par un flexible de raccordement (30) audit au moins un connecteur, sachant que les connecteurs peuvent être reliés aux raccords dans un ordre quelconque et sachant que le dispositif comprend des moyens à l'aide desquels le processus de préparation de la solution de dialyse est exécutable sur la base des moyens de marquage (20) détectés à l'aide du dispositif de saisie.

2. Appareil de dialyse selon la revendication 1, **caractérisé en ce que** des sachets de solution sont prévus comme récipients de réserve et **en ce que** les sachets de solution comprennent le flexible de raccordement (30) à l'extrémité duquel est prévu un connecteur (10).

3. Appareil de dialyse selon la revendication 1 ou 2, **caractérisé en ce que** le moyen de marquage (20) comporte un code à barre (22).

4. Appareil de dialyse selon l'une ou plusieurs des revendications 1 à 3, **caractérisé en ce qu'**il est prévu une unité de dépouillement qui peut être reliée au dispositif de saisie, sachant que l'unité de dépouillement est exécutée de telle sorte que des valeurs de consigne pour la quantité et/ou la nature des composants de la solution pouvant être introduits dans les récipients de réserve peuvent être enregistrées et peuvent être comparées aux valeurs réelles déterminées par le dispositif de saisie.

5. Appareil de dialyse selon la revendication 4, **caractérisé en ce qu'**il est prévu des moyens de signalisation ou de fermeture qui peuvent être reliés à l'unité de dépouillement.

6. Appareil de dialyse selon la revendication 5, **caractérisé en ce que** les moyens de fermeture comprennent des moyens pour la fermeture mécanique et/ou électrique de conduits.
